Europäisches Patentamt

European Patent Office

Office européen des brevets

(19)

(11) Publication number: **0 295 305**
A1

(12)

# EUROPEAN PATENT APPLICATION

published in accordance with Art. 158(3) EPC

(21) Application number: 87906106.7

(22) Date of filing: **18.09.87**

Data of the international application taken as a basis:

(86) International application number: **PCT/JP 87/00690**

(87) International publication number: **WO 88/02006 (24.03.88 88/7)**

(51) Int. Cl.⁴: **C 07 K 15/04**, C 12 N 5/00, C 12 N 15/00, C 12 P 21/00 // A61K39/395, G01N33/574, G01N33/577 ,(C12P21/00, C12R1:91)

(30) Priority: **19.09.86 JP 221390/86**

(43) Date of publication of application: **21.12.88 Bulletin 88/51**

(84) Designated Contracting States: **DE FR GB**

(71) Applicant: **MEIJI MILK PRODUCTS COMPANY LIMITED, 3-6, Kyobashi 2-chome Chuo-ku, Tokyo 104 (JP)**

(72) Inventor: **TADA, Nobuhiko, 793, Itabashi Aza Kaminoyama, Odawara-shi Kanagawa-ken 250 (JP)**
Inventor: **NAKAMURA, Shuji, 2-39-10, Minamikamonomiya, Odawara-shi Kanagawa-ken 250 (JP)**
Inventor: **IKEGAMI, Shuji, Seiryuso 1-212 1123, Kayama, Odawara-shi Kanagawa-ken 250-01 (JP)**

(74) Representative: **Kohn, Armand, 5 Avenue Foch, F-92380 Garches (FR)**

(54) **MONOCLONAL ANTIBODY SPECIFIC TO TUMOR CELL SURFACE GANGLIOSIDE AND HYBRIDOMA YIELDING SAME.**

(57) Monoclonal antibody specifically recognizing a ganglioside present on the surface of mouse and human tumor cells and having a cytotoxic activity against the tumor cells, and a hybridoma yielding the same. The monoclonal antibody is yielded by fused cells composed of antibody-yielding cells and myeloma cells of mice sensitized with lymphoid tumor cells of mice. The monoclonal antibody specifically binds with GD2 of a ganglioside present on the cell surface to exert cytotoxicity, thus being useful for diagnosis and treatment of tumor.

EP 0 295 305 A1

SPECIFICATION

Title of the Invention

ANTI-TUMOR CELL SURFACE GANGLIOSIDE-SPECIFIC
MONOCLONAL ANTIBODY AND HYBRIDOMA PRODUCING THE
SAME

Field of the Industrial Application

The present invention relates to novel monoclonal
antibodies and hybridomas producing the same. More
particularly, the present invention relates to novel
hybridomas obtained by cloning fused cells of mouse
antibody-producing cells sensitized with mouse lymphoid
tumor cells and mouse myeloma cells and characterized
by producing monoclonal antibodies which specifically
recognize gangliosides, in particular, GD2 (GalNAcβl →
4 (NeuAcα2 → 8NeuAcα2 → 3) Galβl → 4Glc → Cer),
which are tumor-related antigens on the cell surface
common to interspecies of mouse and human tumor cells
and having a cytotoxic activity against the tumor
cells, as well as the monoclonal antibodies.

Herein names (GD2, GD3, etc.) of the gangliosides
are given according to the Svennerholm nomenclature
(Svennerholm, L.J., Lipid Res. 5, 145-155 (1964)).
Sialic acid derivatives of GD3 are given within
parenthesis. Namely, GD3 (NeuAc, NeuAc), GD3 (NeuAc, NeuGc), GD3 (NeuGc, NeuAc) and GD3 (NeuGc, NeuGc) mean $11^3(\text{NeuAc})_2$-LacCer, $11^3(\text{NeuAc}\alpha 2 \longrightarrow 8\text{NeuGc})_2$-LacCer, $11^3(\text{NeuGc}\alpha 2 \longrightarrow 8\text{NeuAc})_2$-LacCer and $11^3(\text{NeuGc})_2$-LacCer, respectively. Names of the other substances are given according to the IUPAC-IUB Conference (IUPAC-IUB Commission on Biochemical Nomenclature, Lipids, 12, 455-463 (1977)).

Prior Art

An attempt to analyze human tumor antigens using monoclonal antibodies has begun immediately after the hybridoma method was established. Production of anti-human melanoma monoclonal antibody has been reporeted by H. Koprowski et al. as early as 1978 (H. Koprowski et al., Proc. Natl. Acad. Sci. USA, 75, 3405 (1978)). A great attention was brought to the report on this anti-malignant tumor antibody in the clinical field of diagnosis and treatment of cancers in view of a possibility that it specifically recognized tumor-specific antibody. Thereafter similar investigations were made by many researchers energetically (Proc. Natl. Acad. Sci. USA, 76, 1438 (1979), Proc. Natl. Acad. Sci. USA, 77, 6891 (1980), Br. J. Cancer, 43, 696 (1981)).

- 3 -      0295305

At the beginning, such investigations were made on tumor cell proteins as antigen determinants of tumor antigens and monoclonal antibodies thereto were produced. However, many of them was reactive with normal cells but not satisfactory in specificity to tumor cells. As examination went on antibodies having a relatively high specificity to tumor cells among the monoclonal antibodies obtained, it has been gradually clarified by results of biochemical analysis that many of these antibodies recognize the sugar chain site of complex sugars such as glycolipids or glycoprpteins on the surface of tumor cells as the antigen determinant. Inter alia, utility of monoclonal antibodies recognizing glycolipids containing sialic acid at the ends thereof, namely gangliosides (glycolipids containing sialic acid) as antigens has been recently reported (Cancer Res., 45, 2405-2414 (1985)).

A part of the monoclonal antibodies which can recognize the sugar chain of tumor cells represented by such gangliosides has already begun to be applied to serological diagnosis of cancer. For example, N-19-9 antibody found by Koprowski et al. (Science, 212, 53-54 (1981)) has been evaluated to be useful for diagnosis of pancreatic cancer. This antibody was not developed originally as an antibody, aiming at the sugar chain containing sialic acid but as a result of subsequent biochemical analyse, it was found that the antibody recognized the sugar chain containing sialic acid. In addition thereto, antibodies with which usefulness in serological diagnosis of cancers has been confirmed are mostly those capable of specifically recognizing the sugar chain containing sialic acid to date (Kannagi, Journal of the Medical Association, Japan, 95, No. 2, 200-208 (1986), Kannagi, Metabolism, 23, 141-154 (1986)). With the progress of such biochemical analyses, importance of surface sugar chains of tumor cells represented by gangliosides as tumor antigens is increasing. Speaking of monoclonal antibodies thereto, however, it is under that actual situation that monoclonal antibodies to most of the gangliosides have not been produced, in spite that a number of various characteristic gangliosides which are long or short are present in tumor tissues.

With regard to monoclonal antibodies to gangliosides, there are reported on GD3 by Pukel C.S. et al., J. Exp. Med., 155, 1133-1147 (1982) or Nudelman, E. et al., J. Biol. Chem., 257, 12752-12756 (1982); on GD2 by Cahan, L.D. et al., Proc. Natl. Acad.

Sci. USA, 79, 7629-7633 (1982), Cheresh, D.A. et al.,

Proc. Natl. Acad. Sci. USA, 81, 5767-5771 (1984), Cheung, N.K. et al., Cancer Res. 45, 2642-2649 and Saito, M. et al., Biochem. Biophys. Res. Coomun., 127, 1-7 (1985); on GM2 by Tai, T. et al., Proc. Natl. Acad.

Sci. USA, 80, 5392-5396 (1983) or Natoli, E.J. et al.,

Cancer Res., 46, 4116-4120 (1986); on GM3 by Hirabayashi, Y. et al., J. Biol. Chem., 260, 13328-13333 (1985). These findings all indicate that anti-ganglioside monoclonal antibodies react with neuroectodermal origin tumors such as melanoma, neuroblastoma and glioma but do not react with normal cells. And most of hybridomas capable of producing these monoclonal antibodies are produced by the method of hetero- immunization in which mouse antiboy-producing cells sensitized by human tumor cells are fused to mouse myeloma cells. However, most of the hybridomas obtained by this method produce monoclonal antibodies that recognize HLA of human major histocompatibility complex but a very few hybridomas produce the aimed antibodies specific to tumor cell surface gangliosides, which results in extremely complicated operations for cloning.

Accordingly, development of antibodies having a high specificity to the tumor cell surface gangliosides as tumor antigens has not been advanced also due to complicated operations, although it is an important subject of research. The development is an important subject to be studied from now on for diagnosis and treatment of tumors in the clinical field.

## Means for solving the Problem

Therefore, the present inventors have made investigations, aiming at producing monoclonal antibodies specifically reacting with gangliosides of tumor cells and have found monoclonal antibodies, from hybridomas obtained by cloning fused cells of mouse antiboy-producing cells sensitized with mouse lymphoid tumor cells and mouse myeloma cells, which specifically recognize gangliosides which are tumor-related antigens on the cell surface common to interspecies of mouse and human tumor cells, in particular, GD 2 and having a cytotoxic activity on the tumor cells and, have thus come to accomplish the present invention. It is also a great characteristic that upon production of the hybridomas of the present invention, allogenic mouse lymphoid tumor cells are used as antigens to sensitize mouse so that hybridomas capable of producing the objective monoclonal antibodies can be obtained in a much higher probability than in the conventional method using heterogenic human tumor cells as antigens.

Description of the Drawings

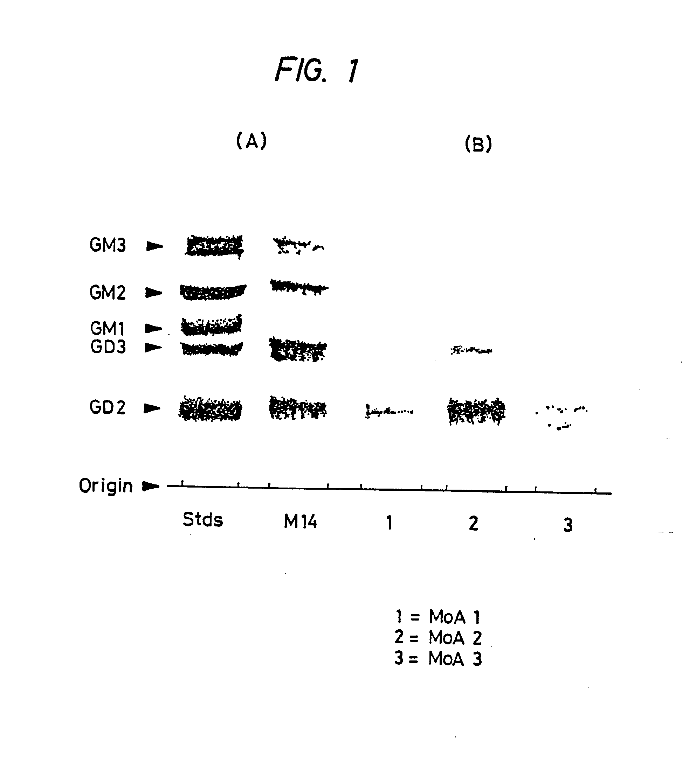

Figure 1 (A) shows, from the left, a mixture of standard gangliosides (GM3 + GM2 + GM1 + GD3 + GD2) and a ganglioside fraction of M14 which are subjected to TLC and stained with resorcinol. Figure 1 (B) shows results obtained after subjecting M14 to TLC and performing enzyme immunostaining with MoA1, MoA2 and MoA3.

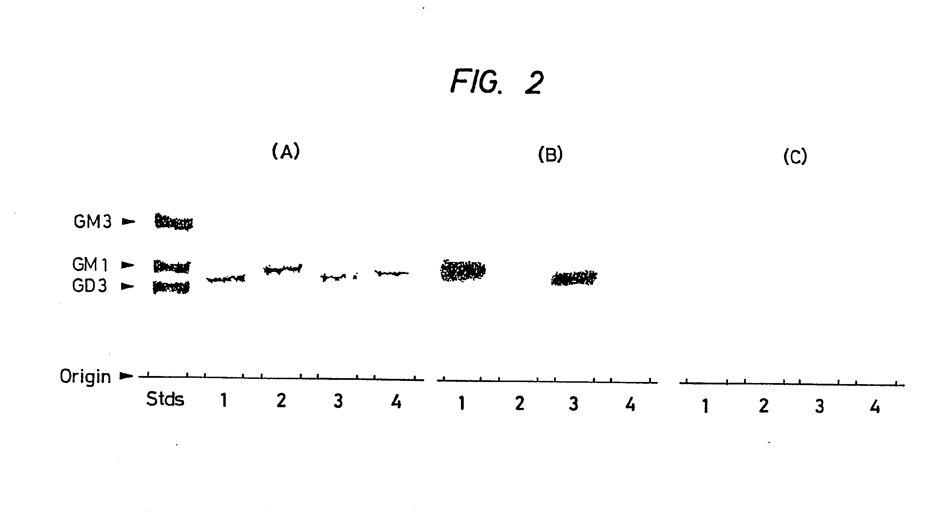

Figure 2 (A) shows 4 kinds of GD3 which are subjected to TLC and stained with resorcinol, indicating from the left a mixture of pure gangliosides (GM3 + GM1 + GD3), lane No. 1 being GD3 (NeuAc, NeuAc), lane No. 2 being GD3 (NeuAc, NeuGc), lane No. 3 being GD3 (NeuGc, NeuAc) and lane No. 4 being GD3 (NeuGc, NeuGc). Figure 2 (B) and (C) show results obtained after performing enzyme immunostaining with MoA2 and MoA1 on TLC, respectively. Lane numbers and kind of GD3 are the same as in Figure 2 (A).

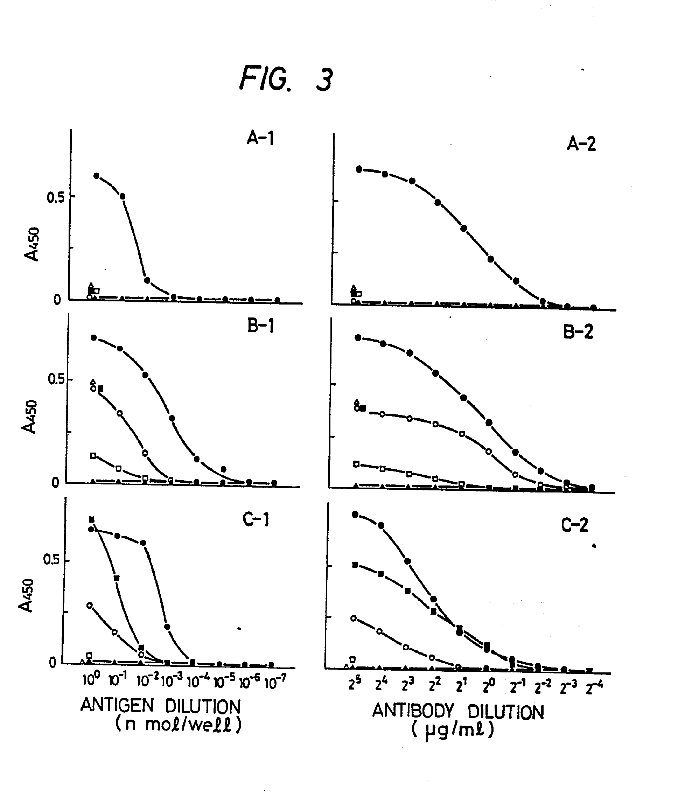

Figure 3 shows results obtained by measurement of reactivity of MoA1, MoA2 and MoA3 with standard gangliosides by ELIZA. In each graph, ● - ●, o - o, ▵-▵, ☐ - ☐, ■ - ■ and ▲ - ▲ indicate GD2, GD3 (NeuAc, NeuAc), GD3

(NeuGc, NeuAc) and GT1a, GD1b, GT1b, GQ1b and, GD3 (NeuAc, NeuGc), GD3 (NeuGc, NeuGc), GD1a, monosialosyl-ganglioside and neutral glycolipid, respectively. Figure 3 (A-1) shows results obtained by serially diluting each standard ganglioside of antigens, coating a well with dilution, reacting with MoA1 (10 µg/ml) and further reacting with enzyme-labeled anti-mouse IgG antibody. Figure 3 (A-2) shows results obtained by adding serially diluted MoA1 to a definite amount of ganglioside (0.1 µg/well) followed by operations similar to (A-1). Figure 3 (B-1) and (B-2) show results obtained on MoA2 by performing operations similar to Figure 3 (A-1) and (A-2), respectively. Figure 3 (C-1) and (C-2) show results obtained on MoA3 by performing operations similar to Figure 3 (A-1) and (A-2), respectively.

Construction of the Invention

The monoclonal antibodies of the present invention specifically distinguish and specifically recognize gangliosides on the surface of tumor cells embryologically classified into tumor cells derived from human neural crest, from normal cells; phrased in more detail, they react with GD2 in common and most potently, which is one of the gangliosides to be recognized.

The monoclonal antibodies of the present invention are produced by culturing the hybridomas obtained by cloning the fused cells of mouse antibody-producing cells sensitized with mouse lymphoid tumor cells and mouse myeloma cells.

In the hybridomas of the present invention, mice are firstly sensitized with mouse lymphoid tumor cells. Any mouse lymphoid tumor cells can be used as far as they are allogenic to mice to be sensitized and established but it is desired that their cytological properties be relatively well studied. Upon sensitization of mice with mouse lymphoid tumor cells, immunization is effected by inoculating intraperitoneally or subcutaneously several times every or every other week in $10^6$ to $10^7$ cells/mouse.

One to five days after final immunization, spleen is removed and its cells are fused as antibody-producing cells to parent cells to give hybridomas.

As the parent cells, mouse myeloma having an adequate selection marker is appropriate.

In production of the hybridomas, mouse myeloma as parent cells and antibody-producing cells are prepared in suitable ratios and a conventional method using a fusing agent is applied. It is suitable to use polyethylene glycol or the like as the fusing agent in a ratio of 1 : 3 to 1 : 5.

From the thus obtained hybridomas, a stable single hybridoma is selected by repeating cloning according to the limiting dilution method which is a known method. Then, the hybridoma producing the antibody of the present invention can be obtained by checking production of an antibody having cytotoxicity against the objective tumor cells.

Such screening of the hybridoma in production of antibody is carried out by either the direct method cytotoxicity test that is a known method using mouse T cell line EL4 and rabbit serum complement of good quality in the culture supernatant obtained by culturing the hybridoma or the immune adherence test using neural crest-derived human tumor cells, diluted guinea pig serum complement and complement receptor of human O-type erythyrocyte in the culture supernatant.

For production of the monoclonal antibody using the thus obtained hybridoma of the present invention, the hybridoma can be cultured, if necessary, utilizing a culture container (in vitro) or in the abdominal cavity (in vivo) of animals to obtain the monoclonal antibody having a high antibody titer. In the case of in vitro, it is sufficient to use an ordinary medium supplemented with FCS and in the case of in vivo, the hybridoma is inoculated in the abdominal cavity of nude mouse and ascites can be collected 7 to 14 days after.

Purification of the monoclonal antibody of the present invention which specifically recognizes the ganglioside of tumor cells on the surface thereof can be carried out by suitably combining known methods such as ammonium sulfate fractionation, gel filtration, affinity column chromatography, etc.

According to the present invention, 3 monoclonal antibodies having similar specificity and hybridomas are obtained in such a manner.

The thus obtained antibodies specifically react with embryologically human neural crest-derived cells changed to tumors, represented by human glioma, human neuroblastoma, human melanoma, etc. but do not react with normal cells. This specificity is common to human neural crest-derived tumor cells and a part of mouse-derived tumor cells. Further the antibodies specifically recognize, as antigenic determinants of human neural crest-derived tumor cells, the ganglioside on the surface of human neural crest-derived tumor cells, which is confirmed by the results of inhibition of immune adherence to heat-treated neural crest-derived tumor cells, disappearance of reactivity with tumor cells treated by sialidase or inhibition of immune adherence to ganglioside fractions.

Further in order to determine the fine structure recognized by these monoclonal antibodies, enzyme immunostaining, enzyme-linked immunosorbent assay (hereafter referred to as ELIZA), IA inhibition test using standard glycolipids were performed on thin layer chromatography (hereafter referred to as TLC); it was noted that the 3 monoclonal antibodies showed a slight difference in gangliosides to be recognized but recognized ganglioside GD2 in common and most potently.

It is expected that the thus obtained monoclonal antibodies which specifically react with human neural crest-derived tumor cells are utilizable for serological test, tissue diagnosis, diagnosis of tumor by imaging, etc.

Hereafter the present invention will be more concretely described by referring to examples below.

Example 1  Production of Hybridoma

Tumor cell line EL4, derived from C57BL mouse T cell, was inoculated in the abdominal cavity of A mice once a week for 4 weeks in $10^7$ cells/mouse and the spleen was ectomized on Day 3 after final inoculation and prepared into a cell suspension.

On the other hand, BALB/c mouse-derived myeloma NS. 1 was prepared and cultured in $10^7$ cells as parent cells of fusion.

Fusion was carried out by the known method (Nature, 256, 495 (1975)) as follows.

The mouse spleen cells and myeloma cells prepared as described above were mixed in a proportion of 5 : 1 in a centrifugal tube. After centrifugation, the supernatant was discarded and to the residue 0.2 ml of polyethylene glycol (mean molecular weight of 4,000, manufactured by Merck Inc.) adjusted to 40% with RPMI 1640 (manufactured by GIBCO Co., Ltd.) was added, followed by thorough mixing for 2 minutes. Then, 5 ml of medium was dropwise added over 3 minutes to dilute. After entrifugation (500 rpm, 5 min), the supernatant was discarded. Next, the cells were suspended in HAT medium (RPMI 1640 medium supplemented with, as final concentrations, $10^{-4}$M of hypoxanthine, $4 \times 10^{-7}$M of aminopterine and

1.6 x $10^{-5}$M of thymidine in 10% FCS) and the suspension was distributed in a 96-well microplate by about 100 µl/well each followed by incubation.

On Day 3 and Day 6 of the incubation, the medium was exchanged with HAT medium and after that, the medium was exchanged with HT medium (medium having removed aminopterine alone from the composition of HAT medium) every 3 other day.

When the formation of colonies could be sufficiently observed (10 to 14 days after), the antibody activity in the culture supernatant was screened by the direct cytotoxicity test using rabbit serum complement of good quality.

The direct cytotoxicity test was carried out by mixing 20 1 of C57BL mouse-derived T cell tumor cell line EL4 suspension, 20 µl of rabbit serum complement and 20 µl of the culture supernatant at 37°C for 45 minutes and examining the presence or absence of cytotoxicity.

Among 486 hybridomas screened, 14 hybridomas that reacted with EL4 cells were obtained. With 3 hybridomas among them, limiting dilution was carried out using a 96-well microplate and cloning was repeated to obtain stable clones. Thus stable and single hybridomas A1, A2 and A3 were obtained.

Immunoglobulin class of a monoclonal antibody produced by each hybridoma was determined by the Ouchterlony's method, whereby monoclonal antibodies produced by A1, A2 and A3 were (IgM,$\chi$), (IgM,$\chi$) and (IgG3,$\chi$), respectively.

Example 2 Purification of monoclonal antibody

Bristen was intraperitoneally administered to three BALB/c nude mice in a dose of 0.5 ml and 7 days after, about 5 to 10 x $10^6$ cells of A1, A2 and A3 were inoculated in the previously prepared nude mice, respectively to cause ascites. The ascites was collected 1 to 2 weeks after and the upper Bristen layer and cell mass were removed by centrifugation at 3,000 rpm for 15 minutes to recover and store an intermediate layer.

The purification of monoclonal antibody component was carried out by the following procedure.

Ammonium sulfate was added to 10 ml of the ascites solution in a final concentration of 50% followed by salting out and centrifugation at 10,000 rpm for 30 minutes. The precipitates were dissolved in 10 ml of PBS buffer (0.01 M phosphate buffer, containing 0.15 M NaCl, pH 7.2) and dialysis was repeated 3 times against 1 liter of the buffer to give each immunoglobulin fraction.

The thus obtained immunoglobulin fractions of Al and A2 were loaded on a Sephadex G-200 column equilibrated with PBS buffer (0.01 M phosphate buffer, 0.15 M NaCl, pH 7.2). The intially eluted fraction (void fraction) was recovered and made purified IgM antibody and the obtained monoclonal antibodies were made MoAl and MoA2.

Then, the immunoglobulin fraction of A3 was thoroughly dialyzed against 0.04 M phosphate buffer (0.03 M NaCl, pH 8.0). Then, the fraction was loaded on an ion exchange resin DE52 column (manufactured by Whatman Co., Ltd.) equilibrated with the same buffer and non-adsorbed portion to DE52 was recovered. The fraction dialyzed against PBS buffer was made pure IgG antibody and the obtained monoclonal antibody was made MoA3.

Example 3  Examination of specificity of monoclonal antibody

(a) With respect to purified MoAl, MoA2 and MoA3, reactivity with various tumor cell lines was examined. The reactivity test with mouse tumor cell line and the reactivity test with human tumor cell line were carried out by the direct method cytotoxicity test using rabbit serum complement of good quality and the immune adherence test using diluted guinea pig serum complement and complement receptor of human O-type erythrocyte, respectively. The results are shown in Table 1 and Table 2. In the mouse tumor cell line, MoA1 reacted with EL4 cells and RBL5 cells and, MoA2 and MoA3 reacted with RBL5 cells and cells. In the human tumor cell line, MoA1 and MoA3 reacted with cell lines from glioma, melanoma and lung cancer and, MoA2 reacted additionally with the cell line from neuroblastoma, in test with 60 kinds of cell lines. As a result, it was confirmed that MoA1, MoA2 and MoA3 all reacted with human neural crest-derived tumor cell lines and a part of mouse tumor cell lines.

Table 1.  Test on Reactivity with Mouse Tumor Cell Line

| Mouse Tumor Cell Line | Origin | Reactivity | | |
|---|---|---|---|---|
| | | MoA1 | MoA2 | MoA3 |
| EL 4 | T cell | + | + | + |
| RBL 5 | T cell | + | + | + |
| BPC 4 | T cell | − | + | + |
| EMG 2 | myeloma | − | − | − |
| ERLD | T cell | − | − | − |
| 70Z/3 | B cell | − | − | − |
| 416 B | stem cell | − | − | − |
| 8057 | megakaryocytic leukemia | − | − | − |
| 8072 | chronic myelocytic leukemia | − | − | − |
| RLM 1 | T cell | − | − | − |
| BALBRV1 | T cell | − | − | − |
| BALBRVA | T cell | − | − | − |
| LSTRA | Moloney leukemia | − | − | − |
| 18-4 | B cell | − | − | − |
| CMS-4 | fibrosarcoma | − | − | − |
| CMS17 | fibrosarcoma | − | − | − |
| C14 | sarcoma | − | − | − |
| B6RV1 | T cell | − | − | − |
| L1210 | B cell | − | − | − |
| P815 | mastocytoma | − | − | − |

Table 2. Test on Reactivity with Human Tumor Cell Line

| Cancer Tissue from which Human Tumor Cell Line has been Derived | Reactivity | | |
|---|---|---|---|
| | MoA1 | MoA2 | MoA3 |
| Glioma | 9/14 | 13/14 | 5/14 |
| Melanoma | 1/2 | 2/2 | 1/2 |
| Lung cancer | | | |
| Small cell | 2/9 | 1/7 | 1/8 |
| Squamous cell carcinoma | 0/4 | 2/4 | 0/4 |
| Adenocarcinoma | 0/5 | 2/5 | 1/6 |
| Other lung cancers | 0/2 | 0/2 | 0/2 |
| Neuroblastoma | 0/3 | 2/3 | 0/3 |
| Gastric cancer | 0/6 | 0/2 | 0/6 |
| Colonic cancer | 0/2 | 0/2 | 0/2 |
| Pancreatic cancer | 0/1 | 0/1 | 0/1 |
| Liver cancer | 0/1 | 0/1 | 0/1 |
| Mammary cancer | 0/2 | 0/2 | 0/2 |
| Urinary bladder cancer | 0/2 | 0/1 | 0/2 |
| Sarcoma | 0/2 | 1/3 | 0/2 |
| Pharyngeal cancer | 0/1 | 0/1 | 0/1 |
| Hematopoietic tumor | 0/6 | 0/6 | 0/6 |
| Kidney cancer | n.d. | 0/4. | n.d. |

(b) The reactivity with normal cells were carried out similarly. MoAl, MoA2 and MoA3 all did not react with mouse normal cells. The results are shown in Table 3.

<div align="center">Table 3</div>

| Cell | Reactivity | | |
|------|------|------|------|
| | MoAl | MoA2 | MoA3 |
| Thymocyte | - | - | - |
| Spleen cell | - | - | - |
| Lymph node cell | - | - | - |
| Bone marrow cell | - | - | - |

Example 4  Examination on antigenic determinant recognized by monoclonal antibody

In order to determine the antigenic determinant recognized by MoAl, MoA2 and MoA3, immune adherence test (hereafter referred to as IA), IA inhibition test and IA adsorption test were carried out using human melanoma cell line UCLASO-Ml4 (hereafter referred to as Ml4) as a target.

(1)  Method for IA

Human albumin-containing veronal buffer (hereafter referred to as HAVB) having the following composition was previously prepared.

| | | |
|---|---|---|
| Human serum albumin (The Green Cross Corporation) | 4 | ml |
| 0.03 N CaCl$_2$ | 2.5 | ml |
| 0.1 M MgCl$_2$ | 2.5 | ml |
| 20 mg/ml KCl | 5 | ml |
| 0.1 g/ml Glucose | 5 | ml |
| Veronal buffer (x 5) | 100 | ml |

(25 mM barbital, pH 7.5, 0.73 M NaCl)

M14, 25 µl of 1 x 10$^6$ cells/ml and 100 µl of antibody solution were added and reacted at 37°C for 90 minutes while shaking.

To the reaction solution was added 0.8 ml of HAVB followed by centrifugation at 1,400 rpm for 5 minutes. The supernatant was discarded and this operation was repeated 3 times to wash the cells.

Next, 25 µl of guinea pig complement diluted to 1/32 was added thereto and the reaction was carried out at 37°C for 35 to 40 minutes while shaking.

Subsequently, 25 μl of human O-type erythrocyte in $8 \times 10^7$ cells/ml was added thereto followed by reacting at 37°C for 10 minutes while shaking. The reaction was carried out by settling for last 20 minutes.

(2) Determination of thermostable antigen by IA adsorption test

Two suspensions of M14 of $5 \times 10^6$ cells in 0.1 ml of medium were prepared; one was heat treated at 100°C for 5 minutes.

To each suspension was added 0.1 ml of MoA1 antibody solution followed by adsorption reaction at 4°C overnight.

After the reaction, centrifugation was performed at 2,000 rpm for 10 minutes and 0.1 ml of the supernatant was provided for the IA adsorption test according to the IA method as described earlier.

Likewise, MoA2 and MoA2 were also reacted.

As a result, any antibody showed rectivity and it was shown that the binding site was a thermostable antigen, namely, sugar antigen.

(3)  Reaction with sialidase-treated tumor cells

One milliliter of M14 in $1 \times 10^6$ cells/ml was taken and 1 unit of sialidase (manufactured by Sigma Co., Ltd.) was added thereto followed by reacting at 37°C for 2 hours.  Then, the cells were washed with medium and this was used as sialidase-treated cells.

Using the sialidase-treated cells as a target, the reactivities of MoA1, MoA2 and MoA3 were examined by the IA method as described previously; the reactivities all disappeared.

From the foregoing, it was confirmed that any of the antibodies contain sialic acid in the antigenic determinants.

(4)  IA inhibition test using ganglioside fraction

Purification from M14 was carried out by extraction of the total lipid fraction, DEAE chromatography, treatment with alkali and iatrobeads column chromatography, in a manner similar to the Svennerholm et al. method (Biochem. Biophys. Acta, 617, 97-107 (1980)).

After the purified ganglioside fraction was added to the antibody solution in 5 nmols or 10 nmols, IA with M14 was carried out.

As a result, IA reaction was inhibited in all of MoAl, MoA2 and MoA3.

Further after the structure of sialic acid was destroyed by treating the ganglioside fraction with 1% acetic acid at 100°C, it was added to each antibody solution and the IA test was carried out; IA test was inhibited in all of MoAl, MoA2 and MoA3.

From the foregoing, it was confirmed that the antigenic determinants against the antibodies were the tumor cell gangliosides in which sialic acid takes part.

Example 5   Examination on fine structure of antigen determinant to monoclonal antibody

Further in order to determine the fine structure recognized by these monoclonal antibodies, enzyme immunostaining   enzyme-linked immunosorbent method (hereafter referred to as ELIZA), IA inhibition test using standard glycolipids were performed on thin layer chromatography (hereafter referred to as TLC).

(1)   Method for TLC

TLC plate "Silica Gel 60" (thickness 200 μm) pre-coated with silica gel manufactured by Merck Inc. and TLC plastic sheet similarly precoated with silica gel manufactured by Merck Inc. were used.   As a developing solvent for chromatogram, chloroform/methanol/0.22% $CaCl_2$ in water, in a volume ratio of 55/45/10 was used. Resorcinol and orcinol were used to stain the ganglioside and neutral glycolipid developed onto TLC plate, respectively.

(2) Enzyme-linked immunostaining on TLC plate

After completion of TLC of the ganglioside, TLC plate was immersed in a phosphate buffered solution containing 1% bovine serum albumin and 1% polyvinylpyrolidone. After drying in air, the plate was reacted with a monoclonal antibody solution (10 µg/ml) for 2 hours at 25°C. Next, the chromatogram was washed in a phosphate buffered solution (hereafter referred to as PBS) while exchanging PBS 5 times. And horse raddish peroxidase-bound goat anti-mouse IgG and IgM antibodies were reacted with this chromatogram at 25°C for 2 hours. Again while exchanging PBS 5 times, the chromatogram was washed in PBS. Upon staining, citrate-phosphate buffer (pH 5.0, 80 mM) containing 400 µg/ml o-phenylenediamine and 0.12% hydrogen peroxide was used and reacted for 15 minutes. Thereafter immersion in water was performed to discontinue the reaction.

(3)    Preparation of M14 ganglioside fraction and standard glycolipids

The M14 ganglioside fraction was prepared according to Tai, T. et al., Proc. Natl. Acad. Sci. USA, 80, 5392-5396 (1983).

Gangliosides GM1, GD1a, GD1b and GT1b were prepared from the bovine brain and purified according to Kanfer, J.N., Methods Enzymol., 14, 660-664 (1969). CQ1b was purchased from Iatron Co., Ltd. GM3 of human brain was provided by D. L. Svennerholm at Goteborg University in Sweden. GM2 and GD2 were prepared from GM2 and GD1b, respectively, by using bovine testicle ß-galactosidase ( donated from Dr. J.W. Jourdian at Michigan University in U.S.A.). GM4 of human myelin and GT1a of human brain were provided by Dr. Ariga in the Tokyo Metropolitan Institute of Medical Science. GD3 (NeuAc, NeuAc), GD3 (NeuAc, NeuGc), GD3 (NeuGc, NeuAc) and GD3 (NeuGc, NeuGc) of bear erythrocyte were donated from Dr. Hashimoto and Dr. Akimi Suzuki in the Tokyo Metropolitan Institute of Medical Science. LacCer, $GgOs_3Cer$ and $GgOs_4Cer$ were prepared from GM3, GM2 and GM1 by treating them with mild acid and purifying by Iatrobeads column chromatography, respectively. GlcCer was prepared according to Tai, T.

- 27 -

et al., Proc. Natl. Acad. Sci. USA, <u>80</u>, 5392-5396

(1983).  $GbOs_3Cer$ and $GbOs_4Cer$ were purchased from

Supelco Co., Ltd. (Verafonte, PA, U.S.A.).

(4)  Results of enzyme-linked immunostaining on TLC
plate in which the ganglioside of M14 was reacted
with monoclonal antibody

As shown in Figure 1, MoA1 and MoA3 react only
with GD2 of M14 and MoA2 reacts with GD2 and with GD3.

(5)  Results of enzyme-linked immunostaining on TLC
plate in which pure gangliosides were reacted with
monoclonal antibody

The results of enzyme-linked immunostaining of
pure di-, tri- and tetra-sialosyl gangliosides on TLC
plate are shown in Table 4.

Table 4

| Ganglioside | Reactivity | | |
|---|---|---|---|
| | MoA1 | MoA2 | MoA3 |
| GD3 | - | ++ | - |
| GD2 | +++ | +++ | +++ |
| GD1a | - | - | - |
| GD1b | - | ++ | - |
| GT1a | - | ++ | ++ |
| GT1b | - | + | - |
| GQ1b | - | ++ | ++ |
| GT3 | - | - | - |
| GT2 | - | - | - |
| GM4 | - | - | - |
| GM3 | - | - | - |
| GM2 | - | - | - |
| GM1 | - | - | - |
| GlcCer | - | - | - |
| LacCer | - | - | - |
| $GgOs_3Cer$ | - | - | - |
| $GgOs_4Cer$ | - | - | - |
| $GbOs_3Cer$ | - | - | - |
| $GbOs_4Cer$ | - | - | - |

+++ : strong

++ : moderate

+ : weak or trace

− : negative


As is understood from Table 4, any of the monoclonal antibodies of the present invention does not react with monosialosyl-gangliosides (GM4, GM3, GM2 and GM1) and neutral glycolipids (GlcCer, LacCer, GgOs$_3$Cer, GgOs$_4$Cer, GbOs$_3$Cer and GbOs$_4$Cer).

Next, characteristics of the reactivity of each monoclonal antibody will be described below.

MoAl does react only with GD 2 but does not react with other gangliosides. GDlb has a structure that one galactose is added to the structure of GD2 and, GM2 and GD3 are deficient of one N-acetylneuramic acid or one N-acetylgalactosamine from the structure of GD2; in spite, MoAl differentiates GD2 from them and reacts only with GD2.

MoA2 cross-reacts with GD3, GD2, GDlb, GTla, GTlb and GQlb which have tri-saccharide structure of NeuAcα2 → 8NeuAcα2 → 3Gal but does not react with other gangliosides. GD2 shows the strongest reactivity, while GTlb is the weakest. GDlb in which one galactose is added to GD2 shows a poor reactivity and GTlb in which sialic acid is further added to GD1b at the terminal galactose residue shows a poorer reactivity.  From these findings, it is suggested that by sialic acid being added to the terminal galactose residue (GD1b) or terminal sialic acid residue (GT1b), NeuAcα2 $\longrightarrow$ 8NeuAcα2 $\longrightarrow$ 3Gal as the antigenic determinant would be masked.  On the other hand, GQ1b having added sialic acid to the terminal sialic acid residue of GT1b recovers its reactivity to a level of GD1b.  The reactivity of GT1a is similar to GQ1b and therefore, the recognition site of GQ1b should be the terminal tri-saccharide. From the foregoing, it was noted that MoA2 recognized disialosyl group "NeuAcα2 $\longrightarrow$ 8NeuAcα2 $\longrightarrow$ 3" bound to the galactose reasidue (irrespective of inner or terminal site).

MoA3 reacts only with GD2, GT1a and GQ1b.

Next, the results of enzyme immunostaining of 4 kinds of GD3 (GD3 (NeuAc, NeuAc), GD3 (NeuAc, NeuGc), GD3 (NeuGc, NeuAc) and GD3 (NeuGc, NeuGc)) and the monoclonal antibodies on TLC plate are shown in Figure 2.  MoA2 alone reacts with a part of them but MoA1 and MoA3 did not react at all (results of MoA3 are now shown in Figure 2 but the results were similar to MoA1).  GD3 (NeuAc, NeuAc) and GD3 (NeuGc, NeuAc) reacted with MoA2. The results suggest that the inner sialic acid of the trisaccharide would be essential for the recognition of the ganglioside by MoA2 and the sialic acid should be NeuAc residue. When the inner sialic acid is not NeuAc but is NeuGc, no recognition is made. On the other hand, the terminal sialic acid residue is recognized by MoA2, even though it is any of NeuGc and NeuAc. If this is the case, the antigenic determinant of MoA2 should be Siaα2 ⟶ 8NeuAcα2 ⟶ 3Gal residue.

(6) Method for ELIZA

Using a 96-well polystyrene microtiter plate, 50 μl each of glycolipid serially diluted with ethanol was charged in each well. The monoclonal antibody was diluted with PBS containing 1% human serum albumin. As second antibodies, peroxidase-bound goat anti-mouse IgG and IgM antibodies were used.

(7) Results of ELIZA with standard glycolipid

The results are shown in Table 3. Generally speaking, the results are similar to those in the enzyme immunostaining method on TLC plate. However, MoA3 that did not recognize GD3 (NeuAc, NeuAc) and GD3 (NeuGc, NeuAc) in the enzyme immunostaining method on TLC plate reacts in ELIZA.

Further there is a difference in the reactivity of GQ1b between both measurement methods. This is probably because sensitivity would be higher in ELIZA.

(8)  IA adsorption inhibition test

Fifty microliters of various glycolipid antigens serially diluted and 50 μl of antibodies were mixed. The mixture was reacted at 4°C overnight. Thereafter, 25 μl of barbiturate buffer solution containing $3.0 \times 10^4$ of M14 was added thereto and 1A adsorption test was conducted.

(9)  Results of IA adsorption inhibition test

Reactivities of 13 acidic standard glycolipids and 6 neutral standard glycolipids (5 nM) with the monoclonal antibodies of the present invention were examined by the results of IA adsorption inhibition test. The results are shown in Table 5. In the table, the antigen titer represents a reciprocal number of dilution magnification when rosette formation was 50% inhibited.

## Table 5

|  | | Antigen Titer | |
|---|---|---|---|
| Ganglioside | MoA1 | MoA2 | MoA3 |
| GD3(NeuAc, NeuAc) | 0 | 32 | 0 |
| GD3(NeuAc, NeuGc) | 0 | 16 | 0 |
| GD3(NeuGc, NeuAc) | 0 | 0 | 0 |
| GD3(NeuGc, NeuGc) | 0 | 0 | 0 |
| GD2 | 64 | 256 | 256 |
| GD1a | 0 | 0 | 0 |
| GD1b | 0 | 16 | 0 |
| GT1a | 0 | 8 | 2 |
| GT1b | 0 | 2 | 0 |
| GQ1b | 0 | 8 | 8 |

The results are almost similar to those in the enzyme immunostaining on TLC. The bond of MoA1 and M14 was inhibited only with GD2. In the case of MoA2, it was inhibited with 7 gangliosides having a tri-saccharide structure to various degrees. Among them, GD2 showed the maximum inhibition and GT1b showed the minimum inhibition. MoA3 was inhibited with 3 gangliosides of GD2, GT1a and GQ1b. Also in MoA3, GD2 has the maximum inhibitory activity.

These results are summarized as as shown in Table 6.

Table 6

| Monoclonal Antibody | Gangliocide Reacted |
|---|---|
| MoA1 | GD2 |
| MoA2 | GD2 > GD3 (NeuAc, NeuAc), GD3 ( NeuGc, NeuAc), GD1b, GT1a, GQ1b > GT1b |
| MoA3 | GD2 > GT1a, GQ1b |

:

Claims

1.    A monoclonal antibody produced by a fused cell of mouse antibody-producing cell sensitized with mouse lymphoid tumor cell and mouse myeloma cell which specifically recognizes a ganglioside on the cell surface of mouse and human tumor cells and has a cytotoxic activity on said tumor cells.

2.    A monoclonal antibody as claimed in claim 1 wherein said mouse lymphoid tumor cell is T cell leukemia cell line EL4.

3.    A monoclonal antibody as claimed in claim 1 wherein said sensitized mouse is A mouse.

4.    A monoclonal antibody as claimed in claim 1 wherein said human tumor cell is neural crest-derived tumor cell.

5.    A monoclonal antibody as claimed in claim 1 wherein said ganglioside on the cell surface is GD2.

6.    A hybridoma obtained by cloning a fused cell of mouse antibody-producing cell sensitized with mouse lymphoid tumor cell and mouse myeloma cell and capable of producing a monoclonal antibody which specifically recognizes a ganglioside on the cell surface of mouse and human tumor cells and has a cytotoxic activity on said tumor cells.

7. A hybridoma as claimed in claim 6 wherein said mouse lymphoid tumor cell is T cell leukemia cell line EL4.

8. A hybridoma as claimed in claim 6 wherein said sensitized mouse is A mouse.

9. A hybridoma as claimed in claim 6 wherein said human tumor cell is neural crest-derived tumor cell.

10. A hybridoma as claimed in claim 6 wherein said ganglioside on the cell surface is GD2.

113

# FIG. 1

(A)                    (B)

GM3 ►

GM2 ►

GM1 ►
GD3 ►

GD2 ►

Origin ►

Stds    M14    1    2    3

1 = MoA 1
2 = MoA 2
3 = MoA 3

# FIG. 2

(A)          (B)          (C)

GM3 ►

GM1 ►
GD3 ►

Origin ►

Stds    1    2    3    4        1    2    3    4        1    2    3    4

FIG. 3

A-1  A-2  
B-1  B-2  
C-1  C-2

A₄₅₀

ANTIGEN DILUTION
(n mol/well)

ANTIBODY DILUTION
(μg/ml)

# INTERNATIONAL SEARCH REPORT

00295305

International Application No  PCT/JP87/00690

---

**I. CLASSIFICATION OF SUBJECT MATTER** (if several classification symbols apply, indicate all) ³

According to International Patent Classification (IPC) or to both National Classification and IPC

Int.Cl⁴  C07K15/04, C12N5/00, 15/00, C12P21/00//A61K39/395, G01N33/574, 33/577, (C12P21/00, C12R1:91)

---

**II. FIELDS SEARCHED**

Minimum Documentation Searched ⁴

| Classification System | Classification Symbols |
|---|---|
| IPC | C07K15/04, C12N5/00, 15/00, C12P21/00, A61K39/395 |

Documentation Searched other than Minimum Documentation
to the Extent that such Documents are Included in the Fields Searched ⁵

---

**III. DOCUMENTS CONSIDERED TO BE RELEVANT** ¹⁴

| Category * | Citation of Document, ¹⁶ with indication, where appropriate, of the relevant passages ¹⁷ | Relevant to Claim No. ¹⁸ |
|---|---|---|
| A, P | JP, A, 62-190074 (Fred Hatchinson Cancer Research Center) 20 August 1987 (20. 08. 87) (Family: none) | 1-10 |
| A | JP, A, 61-88892 (Shionogi & Co., Ltd.) 7 May 1986 (07. 05. 86) (Family: none) | 1-10 |
| A | Toyama Sakuji and one other (authors) "Mono-Clone Antibody Handbook" Kodansha Kabushiki Kaisha (1985.07.10) P.169, right column, line 3-170. | 1-10 |

---

* Special categories of cited documents: ¹⁵

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

---

**IV. CERTIFICATION**

| Date of the Actual Completion of the International Search ² | Date of Mailing of this International Search Report ² |
|---|---|
| December 4, 1987 (04.12.87) | December 14, 1987 (14.12.87) |

| International Searching Authority ¹ | Signature of Authorized Officer ²⁰ |
|---|---|
| Japanese Patent Office | |

Form PCT/ISA/210 (second sheet) (October 1977)